# EUROPEAN PATENT APPLICATION

(11) **EP 4 197 535 A1**
(43) Date of publication of application: **21.06.2023**
(21) Application number: 22214236.6
(22) Date of filing: 16.12.2022
(51) Int. Cl.: A61K 31/402, A61K 31/4196, A61K 31/426, A61K 31/427, A61K 31/428, A61K 31/4439, A61K 31/454, A61K 31/496, A61K 31/5377, A61K 31/7072, A61P 31/14, A61P 31/16, A61P 31/20

(54) **TREATMENT OF RESPIRATORY VIRUS INFECTION BY MODULATION OF THE N-GLYCOSYLATION PATHWAY**

(30) Priority: 16.12.2021 US 202163290563 P
(71) Applicant: VIR Biotechnology, Inc., San Francisco, CA 94158 (US); Glaxo Wellcome UK Limited, Brentford, Middlesex TW8 9GS (GB)
(72) Inventor: BALCE, Dale R., Millbrae, CA (US); BARTHA, Istvan, Bussigny (CH); GROSSE, Johannes, San Diego, CA (US); HWANG, Seungmin, Saint Louis, MO (US); NEWBY, Zachary, San Francisco, CA (US); PARK, Arnold, Pacifica, CA (US); SORIAGA, Leah B., San Diego, CA (US); TELENTI, Amalio, La Jolla, CA (US); TSE, Winston C., Redwood City, CA (US)
(74) Representative: Mewburn Ellis LLP

(57) **Abstract**

The present disclosure relates to methods of inhibiting replication of a respiratory virus, and methods of treating or preventing a respiratory virus infection in a subject in need thereof comprising administering to the subject a therapeutically effective amount of a *N*-glycosylation pathway inhibitor.

## Description

### CROSS REFERENCE TO RELATED APPLICATIONS

This application claims the benefit of U.S. Provisional Application Serial Number 63/290,563, filed on December 16, 2021, which is hereby incorporated by reference in its entirety.

### BACKGROUND OF THE INVENTION

Respiratory infections are a major cause of morbidity and mortality for humans. A majority of these infections are caused by respiratory viruses. These respiratory viruses include influenza viruses, (e.g., human influenza, epizootic avian influenza viruses such as H5N1, H7N9, and H10N8), coronaviruses (e.g., severe acute respiratory syndrome coronaviruses (SARS-CoV and SARS-CoV2) and Middle East respiratory syndrome coronavirus (MERS-CoV)), human adenovirus-14, human metapneumovirus, enterovirus D68, bunyaviruses, and other common respiratory viruses (respiratory syncytial virus, parainfluenza viruses, and rhinoviruses. The continual emergence of these new and reemerging viral threats underscores the unpredictability of these pathogens, and the challenges inherent in their control. Thus, new treatment and/or prevention modalities for respiratory viruses are needed.

The oligosaccharyltransferase ("OST") complex, localized in the endoplasmic reticulum (ER) of eukaryotic cells, is responsible for the N-linked glycosylation of numerous protein substrates. The membrane protein STT3 is a highly conserved subunit of the oligosaccharyltransferase and contains the active site of the complex. STT3 transfers oligosaccharides onto the asparagine residues of sequons (N-X≠P-T/S/C) in nascent glycoproteins. The two alternate STT3 proteins, STT3A and STT3B are widely expressed in a variety of human tissues and are encoded by different genes. STT3A and STT3B exist in distinct OST complexes, possess different kinetic properties, and have different substrate preferences, in spite of their partially overlapping roles in glycosylation. While the STT3A complex generally promotes co-translational glycosylation, the STT3B complex generally promotes post-translational glycosylation.

### SUMMARY OF THE INVENTION

In certain aspects, the present disclosure provides a method of inhibiting replication of a respiratory virus in a subject in need thereof, the method comprising administering to the subject an *N-*glycosylation pathway inhibitor. In some embodiments, the *N*-glycosylation pathway inhibitor is an OST complex inhibitor. In some embodiments, the OST complex inhibitor is a small molecule OST complex inhibitor. In some embodiments, the *N*-glycosylation pathway inhibitor is selected from tunicamycin and NGI-1. In some embodiments, the *N*-glycosylation pathway inhibitor is tunicamycin. In some embodiments, the *N*-glycosylation pathway inhibitor is NGI-1. In some embodiments, the OST complex inhibitor is STT3A/STT3B inhibitor. In some embodiments, the STT3A/STT3B inhibitor is a small molecule STT3A/STT3B inhibitor. In some embodiments, the STT3A/STT3B inhibitor comprises a protein. In some embodiments, the STT3A/STT3B inhibitor comprises an antibody or antigen binding fragment thereof that selectively binds STT3A, STT3B, or both. In some embodiments, the subject is a human. In some embodiments, the respiratory virus is selected from the group consisting of: influenza virus, a rhinovirus, a coronavirus, a metapneumovirus, an adenovirus, a respiratory syncytial virus, a bocavirus, and a parainfluenza virus. In some embodiments, the respiratory virus is selected from the group consisting of: influenza virus, a rhinovirus, a metapneumovirus, an adenovirus, a respiratory syncytial virus, a bocavirus, and a parainfluenza virus. In some embodiments, the respiratory virus is selected from the group consisting of: an influenza virus, a rhinovirus, and a parainfluenza virus. In some embodiments, the respiratory virus is not a coronavirus. In some embodiments, the respiratory virus is not a SARS-CoV-2 virus. In some embodiments, the coronavirus is a SARS-CoV-2 variant selected from: alpha variant, beta variant, delta variant, and omicron variant. In some embodiments, the method comprises treating a respiratory virus infection in a subject in need thereof. In some embodiments, the method comprises preventing a respiratory virus infection in a subject in need thereof.

In certain aspects, the present disclosure provides a method of treating or preventing a respiratory virus infection in a subject in need thereof, the method comprising administering to the subject an *N*-glycosylation pathway inhibitor. In some embodiments, the *N*-glycosylation pathway inhibitor is an OST complex inhibitor. In some embodiments, the OST complex inhibitor is a small molecule OST complex inhibitor. In some embodiments, the *N*-glycosylation pathway inhibitor is selected from tunicamycin and NGI-1. In some embodiments, the *N*-glycosylation pathway inhibitor is tunicamycin. In some embodiments, the *N*-glycosylation pathway inhibitor is NGI-1. In some embodiments, the OST complex inhibitor is STT3A/STT3B inhibitor. In some embodiments, the STT3A/STT3B inhibitor is a small molecule STT3A/STT3B inhibitor. In some embodiments, the STT3A/STT3B inhibitor comprises a protein. In some embodiments, the STT3A/STT3B inhibitor comprises an antibody or antigen binding fragment thereof that selectively binds STT3A, STT3B, or both. In some embodiments, the subject is a human. In some embodiments, the respiratory virus is selected from the group consisting of: influenza virus, a rhinovirus, a coronavirus, a metapneumovirus, an adenovirus, a respiratory syncytial virus, a bocavirus, and a parainfluenza virus. In some embodiments, the respiratory virus is selected from the group consisting of influenza virus, a rhinovirus, a metapneumovirus, an adenovirus, a respiratory syncytial virus, a bocavirus, and a parainfluenza virus. In some embodiments, the respiratory virus is selected from the group consisting of an influenza virus, a rhinovirus, and a parainfluenza virus. In some embodiments, the respiratory virus is not a coronavirus. In some embodiments, the respiratory virus is not a SARS-CoV-2 virus. In some embodiments, the coronavirus is a SARS-CoV-2 variant selected from: alpha variant, beta variant, delta variant, and omicron variant. In some embodiments, the method comprises treating a respiratory virus infection in a subject in need thereof. In some embodiments, the method comprises preventing a respiratory virus infection in a subject in need thereof.

In certain embodiments, the present disclosure provides an *N-*glycosylation pathway inhibitor for use in inhibiting replication of a respiratory virus in a subject in need thereof. In some embodiments, the *N*-glycosylation pathway inhibitor is an OST complex inhibitor. In some embodiments, the OST complex inhibitor is a small molecule OST complex inhibitor. In some embodiments, the *N-*glycosylation pathway inhibitor is selected from tunicamycin and NGI-1. In some embodiments, the *N*-glycosylation pathway inhibitor is tunicamycin. In some embodiments, the *N*-glycosylation pathway inhibitor is NGI-1. In some embodiments, the OST complex inhibitor is STT3A/STT3B inhibitor. In some embodiments, the STT3A/STT3B inhibitor is a small molecule STT3A/STT3B inhibitor. In some embodiments, the STT3A/STT3B inhibitor comprises a protein. In some embodiments, the STT3A/STT3B inhibitor comprises an antibody or antigen binding fragment thereof that selectively binds STT3A, STT3B, or both. In some embodiments, the subject is a human. In some embodiments, the respiratory virus is selected from the group consisting of: influenza virus, a rhinovirus, a coronavirus, a metapneumovirus, an adenovirus, a respiratory syncytial virus, a bocavirus, and a parainfluenza virus. In some embodiments, the respiratory virus is selected from the group consisting of influenza virus, a rhinovirus, a metapneumovirus, an adenovirus, a respiratory syncytial virus, a bocavirus, and a parainfluenza virus. In some embodiments, the respiratory virus is selected from the group consisting of an influenza virus, a rhinovirus, and a parainfluenza virus. In some embodiments, the respiratory virus is not a coronavirus. In some embodiments, the respiratory virus is not a SARS-CoV-2. In some embodiments, the coronavirus is a SARS-CoV-2 variant selected from: alpha variant, beta variant, delta variant, and omicron variant.

In certain embodiments, the present disclosure provides an N glycosylation pathway inhibitor for use in treating or preventing a respiratory virus infection in a subject in need thereof. In some embodiments, the *N*-glycosylation pathway inhibitor is an OST complex inhibitor. In some embodiments, the OST complex inhibitor is a small molecule OST complex inhibitor. In some embodiments, the *N-*glycosylation pathway inhibitor is selected from tunicamycin and NGI-1. In some embodiments, the *N*-glycosylation pathway inhibitor is tunicamycin. In some embodiments, the *N*-glycosylation pathway inhibitor is NGI-1. In some embodiments, the OST complex inhibitor is STT3A/STT3B inhibitor. In some embodiments, the STT3A/STT3B inhibitor is a small molecule STT3A/STT3B inhibitor. In some embodiments, the STT3A/STT3B inhibitor comprises a protein. In some embodiments, the STT3A/STT3B inhibitor comprises an antibody or antigen binding fragment thereof that selectively binds STT3A, STT3B, or both. In some embodiments, the subject is a human. In some embodiments, the respiratory virus is selected from the group consisting of: influenza virus, a rhinovirus, a coronavirus, a metapneumovirus, an adenovirus, a respiratory syncytial virus, a bocavirus, and a parainfluenza virus. In some embodiments, the respiratory virus is selected from the group consisting of influenza virus, a rhinovirus, a metapneumovirus, an adenovirus, a respiratory syncytial virus, a bocavirus, and a parainfluenza virus. In some embodiments, the respiratory virus is selected from the group consisting of an influenza virus, a rhinovirus, and a parainfluenza virus. In some embodiments, the respiratory virus is not a coronavirus. In some embodiments, the respiratory virus is not a SARS-CoV-2. In some embodiments, the coronavirus is a SARS-CoV-2 variant selected from: alpha variant, beta variant, delta variant, and omicron variant.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### DETAILED DESCRIPTION OF THE INVENTION

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Definitions

Unless defined otherwise, all technical and scientific terms used herein have the same meaning as is commonly understood by one of skill in the art to which this invention belongs. All patents and publications referred to herein are incorporated by reference.

As used in the specification and claims, the singular form "a", "an" and "the" includes plural references unless the context clearly dictates otherwise.

As used herein, the term "antibody or antigen binding fragment thereof' is intended to cover polyclonal antibodies, multiclonal antibodies, monoclonal antibodies, chimeric antibodies, humanized and primatized antibodies, human antibodies, recombinantly produced antibodies, intrabodies, multispecific antibodies, bispecific antibodies, monovalent antibodies, multivalent antibodies, anti-idiotypic antibodies, synthetic antibodies, including muteins and variants thereof; antibody fragments such as Fab fragments, F(ab') fragments, single-chain FvFcs, single-chain Fvs; and derivatives thereof including Fc fusions and other modifications, and any other immunologically active molecule so long as they exhibit the desired biological activity (i.e., antigen association or binding). Moreover, the term further comprises all classes of antibodies (i.e. IgA, IgD, IgE, IgG, and IgM) and all isotypes (i.e., IgG1, IgG2, IgG3, IgG4, IgA1, and IgA2), as well as variations thereof unless otherwise dictated by context.

As used herein, the term "inhibiting replication" includes the reduction in the replication rate of a virus in the presence of a composition, relative to the same virus under comparable conditions in the absence of the composition.

As used herein, the terms "OST complex inhibitor" and "inhibitor of OST complex" includes any agent that inhibits or antagonizes at least one of the proteins and/or subunits of the OST complex. Examples of agents that can be used as inhibitors include, but are not limited to small molecules, nucleic acids, proteins or peptides, and antibodies or antigen binding fragments thereof.

As used herein, the terms "N-glycosylation pathway inhibitor" and "inhibitor of the N-glycosylation pathway" includes any agent that inhibits or antagonizes at least one of the proteins and/or subunits of the N-glycosylation pathway. Examples of agents that can be used as inhibitors include, but are not limited to small molecules, nucleic acids, proteins or peptides, and antibodies or antigen binding fragments thereof.

The term "salt" or "pharmaceutically acceptable salt" refers to salts derived from a variety of organic and inorganic counter ions well known in the art. Pharmaceutically acceptable acid addition salts can be formed with inorganic acids and organic acids. Pharmaceutically acceptable base addition salts can be formed with inorganic and organic bases.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material. Each carrier must be "acceptable" in the sense of being compatible with the other ingredients of the formulation and not injurious to the patient.

The term "preventing" is art-recognized, and when used in relation to a condition, such as an infection by a respiratory virus, is well understood in the art, and includes administration of a composition which reduces the frequency of, or delays the onset of, symptoms of a medical condition in a subject relative to a subject which does not receive the composition. Thus, prevention of respiratory virus infection includes, for example, reducing the number of patients suffering infection by a respiratory virus in a population of patients receiving a prophylactic treatment relative to an untreated control population. Prevention of a respiratory virus infection also includes, for example, reducing the number of diagnoses of the infection in a treated population versus an untreated control population, and/or delaying the onset of symptoms of the infection in a treated population versus an untreated control population

As used herein, the terms "STT3A/STT3B inhibitor" and "inhibitor of STT3A/STT3B" includes any agent that inhibits or antagonizes at least one of the STT3A and/or STT3B subunits. Examples of agents that can be used as inhibitors include, but are not limited to small molecules, nucleic acids, proteins or peptides, and antibodies or antigen binding fragments thereof.

As used herein, the phrase "therapeutically effective amount," means the amount of a compound that, when administered to a patient for treating a disease, is sufficient to treat the disease. The "therapeutically effective amount" will vary depending on the compound, the disease and its severity and the age, weight, adsorption, distribution, metabolism and excretion *etc.,* of the patient to be treated.

As used herein, "treatment" or "treating" refers to an approach for obtaining beneficial or desired results with respect to a disease, disorder, or medical condition including, but not limited to, a therapeutic benefit. In certain embodiments, treatment or treating involves administering a compound or composition disclosed herein to a subject. A therapeutic benefit may include the eradication or amelioration of the underlying disorder being treated.

### N-glycosylation pathway inhibitors and OST complex inhibitors

Provided herein are *N*-glycosylation pathway inhibitors. In some embodiments, the *N-*glycosylation pathway inhibitors are OST complex inhibitors. In some embodiments, the *N-*glycosylation pathway inhibitors may reduce the expression and/or activity of one or more proteins in the N-glycosylation pathway. Exemplary *N*-glycosylation pathway inhibitors include, for example, nucleic acids, proteins, small molecules, or large molecules. In some embodiments, small molecule *N-*glycosylation pathway inhibitors may be identified, for example, using a biochemical assay testing the enzymatic activity of recombinant human proteins of the *N*-glycosylation pathway. In some embodiments, and/or small molecule OST complex inhibitors may be identified, for example, using a biochemical assay testing the activity of one or more recombinant human proteins and/or protein subunits of the OST complex.

In some embodiments, the OST complex inhibitors are STT3A/STT3B inhibitors. Such inhibitors may reduce the expression and/or activity of STT3A and/or STT3B in a cell. Exemplary STT3A/STT3B inhibitors include, for example, nucleic acids, proteins, small molecules, or large molecules. Small molecule STT3A/STT3B inhibitors may be identified using a biochemical assay testing the enzymatic activity of recombinant human STT3A and/or STT3B.

In some embodiments, the STT3A/STT3B inhibitor is a small molecule STT3A/STT3B inhibitor. In some embodiments, the STT3A/STT3B inhibitor is a protein STT3A/STT3B inhibitor. In some embodiments, the STT3A/STT3B inhibitor is an anti-STT3A and/or anti-STT3B antibody or antigen binding fragment thereof. In some embodiments, the STT3A/STT3B inhibitor is a nucleic acid.

In some embodiments, after a STT3A/STT3B inhibitor is contacted with cells, STT3A/STT3B expression and/or activity is inhibited in at least 10%, at least 20%, at least 30%, at least 40%, at least 50%, at least 60%, at least 70%, at least 80%, at least 90%, at least 95%, at least 99%, or 100% of the cells.

In some embodiments, the OST complex inhibitors are small molecule OST complex inhibitors. In some embodiments, the small molecule OST complex inhibitors include those small molecule STT3A/STT3B inhibitors disclosed, for example, in the following publications: N. Rinis, et al., Editing N-Glycan Site Occupancy with Small-Molecule Oligosaccharyltransferase Inhibitors, Cell Chem Biol., 25:1231-1241 (2018); A.S. Puschnik, et al., A Small-Molecule Oligosaccharyltransferase Inhibitor with Pan-flaviviral Activity, Cell Reports, 21:3032-3039 (2017); C. Lopez-Sambrooks, et al., Oligosaccharyltransferase Inhibition Induces Senescence In RTK-Driven Tumor Cells, Nat. Chem. Biol., 12:1023-1030 (2016); and WO 2017/019540 to J.N. Contessa, et al.

In some embodiments, the exemplary small molecule N-glycosylation pathway inhibitor for use in the methods described herein are provided below:
- 5-(N,N-Dimethylsulfamoyl)-N-(5-methy-lthiazol-2-yl)-2-(pyrrolidin-1-yl)benzamide
- 5-(dimethylsulfamoyl)-N-(4-methyl-1,3-thiazol-2-yl)-2-pyrrolidin-1-ylbenzamide
- 5-(dimethylsulfamoyl)-2-pyrrolidin-1-yl-N-(1,3-thiazol-2-yl)benzamide
- 5-(di methyl sulfamoyl)-N-(5-methyl-1,3,4-triadiazol-2-yl)-2-pyrrolidin-1-ylbenzamide
- 5-(dimethylsulfamoyl)-N-(5-methyl-1H-1,2,4-triazol-3-yl)-2-pyrrolidin-1-ylbenzamide
- N-(1,3-benzothiazol-2-yl)-5-(dimethylsulfamoyl)-2-pyrrolidin-1-ylbenzamide
- 5-(dimethylsulfamoyl)-N-(4-methoxy-1,3-benzothiazol-2-yl)-2-pyrrolidin-1-ylbenzamide
- 5-(dimethylsulfamoyl)-N-(5-methoxy-1,3-benzothiazol-2-yl)-2-pyrrolidin-1-ylbenzamide
- 5-(dimethylsulfamoyl)-N-(6-methoxy-1,3-benzothiazol-2-yl)-2-pyrrolidin-1-ylbenzamide
- 5-(dimethyl sulfamoyl)-N-pyridin-3-yl-2-pyrrolidin-1-ylbenzamide
- 5-(dimethylsulfamoyl)-N-pyridin-4-yl-2-pyrrolidin-1-ylbenzamide
- 5-(dimethylsulfamoyl)-N-phenyl-2-pyrrolidin-1-ylbenzamide
- 5-(dimethylsulfamoyl)-N-(2-methylphenyl)-2-pyrrolidin-1-ylbenzamide
- 5-(dimethylsulfamoyl)-N-(4-methylphenyl)-2-pyrrolidin-1-ylbenzamide
- 5-(dimethylsulfamoyl)-N-(2-methoxyphenyl)-2-pyrrolidin-1-ylbenzamide
- 5-(dimethylsulfamoyl)-N-(4-methoxyphenyl)-2-pyrrolidin-1-ylbenzamide
- 5-(dimethylsulfamoyl)-N-(2-fluorophenyl)-2-pyrrolidin-1 -ylbenzamide
- 5-(dimethylsulfamoyl)-N -(3-fluorophenyl)-2-pyrrolidin- 1-ylbenzamide
- 5-(dimethylsulfamoyl)-N-(2-chlorophenyl)-2-pyrrolidin-1-ylbenzamide
- 5-(dimethylsulfamoyl)-N-(4-bromophenyl)-2-pyrrolidin- 1 -ylbenzamide
- N,N-dimethyl-3-(morpholine-4-carbonyl)-4-pyrrolidin-1-ylbenzenesulfonamide
- N-cyclohexyl-5-(dimethylsulfamoyl)-2-pyrrolidin- 1 -ylbenzamide
- 5-(dimethylsulfamoyl)-N,N-dimethyl-2-pyrrolidin- 1-ylbenzamide
- 5-(dimethylsulfamoyl)-N-(5-methyl-1,3-thiazol-2-yl)-2-pyrrolidin-1 -ylbenzamide
- 5-(diethylsulfamoyl)-N-(5-methyl- 1,3-thiazol-2-yl)-2-pyrrolidin- 1-ylbenzamide
- N-(5-methyl-1,3-thiazol-2-yl)-2-pyrrolidin- 1 -yl-5-pyrrolidin- 1 -ylsulfonylbenzamide
- N-(5-methyl-1,3-thiazol-2-yl)-2-piperidine-1 -yl-5-pyrrolidin- 1-ylsulfonylbenzamide
- N-(5-methyl- 1,3-thiazol-2-yl)-2-morpholin- 1 -yl-5-pyrrolidin- 1 -ylsulfonylbenzamide
- N-(5-methyl-1,3-thiazol-2-yl)-2-piperazin-1-yl-5-pyrroli din- 1 -ylsulfonylbenzamide
- 5-[methyl(phenyl)sulfamoyl]-N-(5-methyl-1,3-thiazol-2-yl)-2-pyrrolidin-1-ylbenzamide
- 5-(benzylsulfamoyl)- N-(5-methyl-1,3-thi azol-2-yl)-2-pyrrolidin- 1-ylbenzamide
- 5 -(dimethylsulfamoyl)-N-(5-methyl-1,3-thiazol-2-yl)-2-pyrrolidin-1-ylbenzamide
- 5-(dimethylsulfamoyl)-N-(5-methyl-1,3-thiazol-2-yl)-2-piperidin-1-ylbenzamide
- 2-(azetidin- 1-yl)-5-(dimethylsulfamoyl)-N-(5-methyl- 1,3-thiazol -2-yl)benzamide
- 5 -(dimethylsulfamoyl)-N-(5-methyl-1,3-thiazol-2-yl)-2-piperazin-1-ylbenzamide
- 2-(dimethylamino)-5-(dimethylsulfamoyl)-N-(5-methyl-1,3-thiazol-2-yl)benzamide
- 2-(diethylamino)-5-(dimethylsulfamoyl)-N-(5-methyl-1,3-thiazol-2-yl)benzamide
- 2-cyclopentyl -5-(dimethylsulfamoyl)-N-(5-methyl- 1,3-thiazol-2- yl)benzamide
- 3-(dimethylsulfamoyl)-N-(5-methyl-1,3-thiazol-2-yl)benzamide
- (*E*)-*N*-[(2*S*,3*R*,4*R*,5*R*,6*R*)-2-[(2*R*,3*R*,4*R*,5*S*,6*R*)-3-acetamido-4,5-dihydroxy-6-(hydroxymethyl)oxan-2-yl]oxy- 6-[2-[(2*R*,3*S*,4*R*,S*R*)-5-(2,4-dioxopyrimidin-1-yl)- 3,4-dihydroxyoxolan-2-yl]-2-hydroxyethyl]-4,5-dihydroxyoxan-3-yl]-5-methylhex-2-enamide

In some embodiments, the exemplary small molecule N-glycosylation pathway inhibitor for use in the methods described herein is selected from: 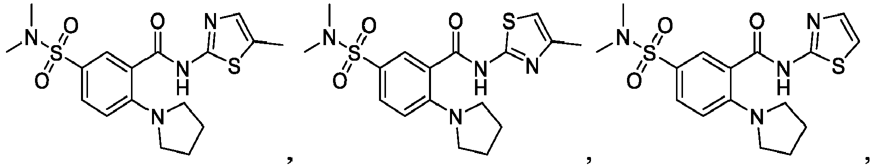 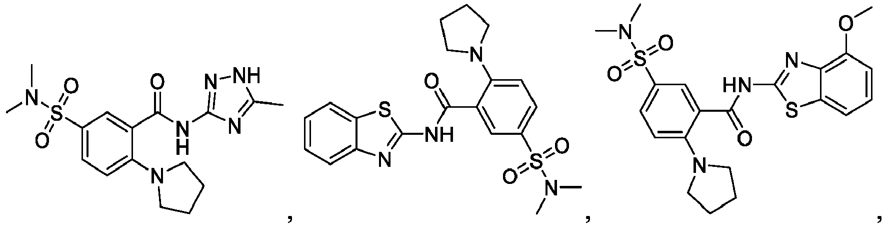 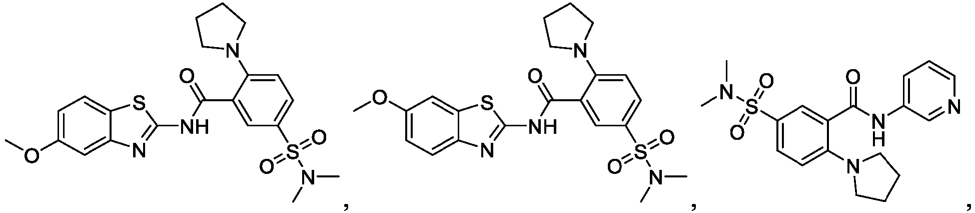 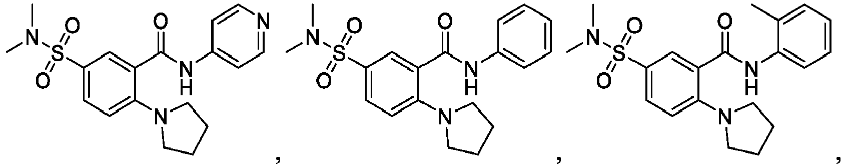 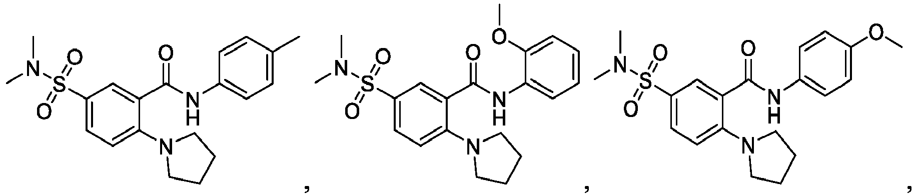 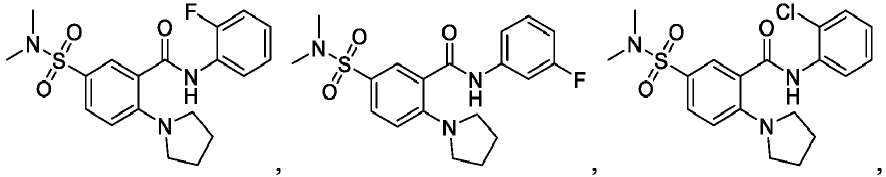 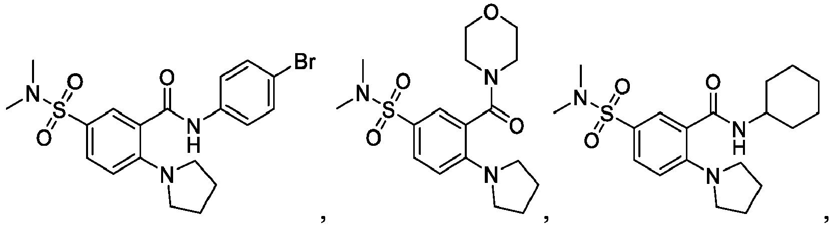 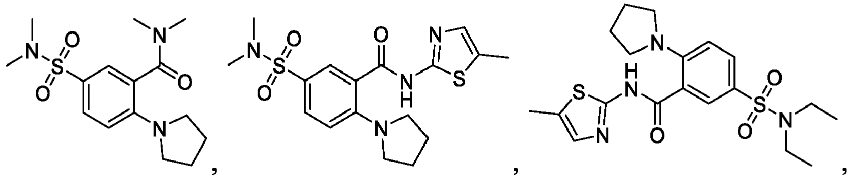 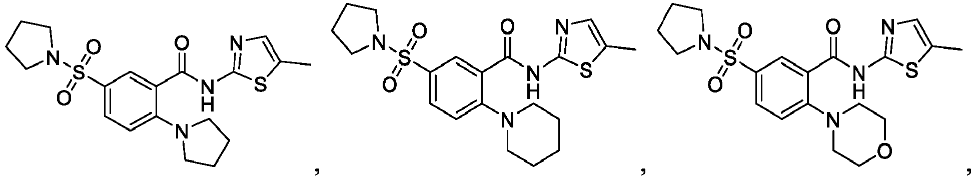 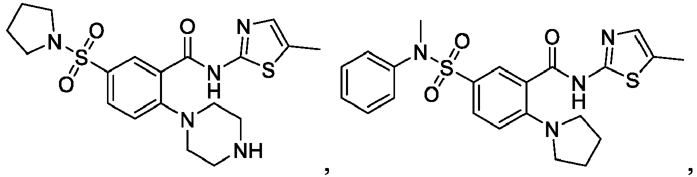 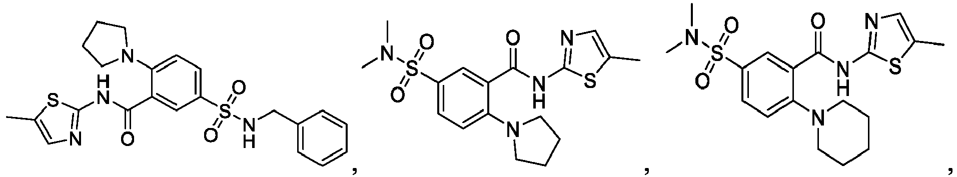 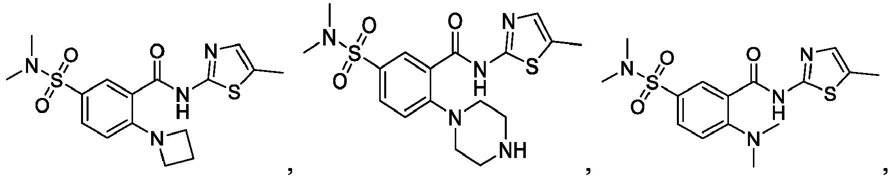 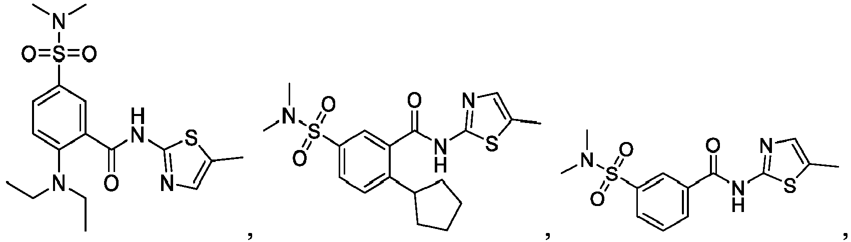 and

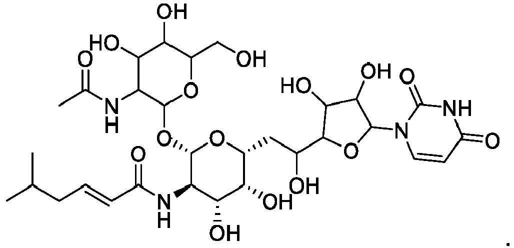

In some embodiments of the present disclosure, the N-glycosylation pathway inhibitor is selected from tunicamycin and NGI-1 (5-(N,N-dimethylsulfamoyl)-N-(5-methylthiazol-2-yl)-2-(pyrrolidin-1-yl)benzamide). In some embodiments of the present disclosure, the N-glycosylation pathway inhibitor is tunicamycin. In some embodiments of the present disclosure, the N-glycosylation pathway inhibitor is NGI-1. In some embodiments of the present disclosure, the N-glycosylation pathway inhibitor is an OST complex inhibitor. In some embodiments, the OST complex inhibitor is NGI-1.

It has been discovered that such small molecule OST complex inhibitors and/or *N-*glycosylation pathway inhibitors are useful for the treatment or prevention of respiratory virus infections. It has further been discovered that such small molecule OST complex inhibitors and/or glycosylation pathway inhibitors are also useful for inhibiting replication of respiratory viruses. In some embodiments the respiratory viral infection is caused by, for example, influenza virus, a rhinovirus, a coronavirus, a metapneumovirus, an adenoviruses, a syncytial virus, a bocaviruses, and a parainfluenza virus.

In some embodiments, the OST complex inhibitors are protein STT3A/STT3B inhibitors. In some embodiments, the STT3A/STT3B inhibitor is an anti-STT3A and/or anti-STT3B antibody or antigen binding fragment thereof. In some embodiments, the anti-STT3A and/or anti-STT3B antibody include, for example, the antibodies disclosed in N.A. Cherepanova, et al, Mammalian cells lacking either the cotranslational or posttranslocational oligosaccharyltransferase complex display substrate-dependent defects in asparagine linked glycosylation, Sci. Reports, 6:20946 (2016).

The *N*-glycosylation pathway inhibitors, OST complex inhibitors, and/or STT3A/STT3B inhibitors described herein can be used in the preparation of medicaments for the prevention or treatment of diseases or conditions. In addition, a method for treating any of the diseases or conditions described herein in a subject in need of such treatment, involves administration of pharmaceutical compositions containing at least one compound described herein, or a pharmaceutically acceptable salt, pharmaceutically acceptable prodrug, or pharmaceutically acceptable solvate thereof, in therapeutically effective amounts to said subject.

The compositions containing the *N-*glycosylation pathway inhibitors, OST complex inhibitors, and/or STT3A/STT3B inhibitors described herein can be administered for prophylactic and/or therapeutic treatments. In therapeutic applications, the compositions are administered to a patient already suffering from a disease or condition, in an amount sufficient to cure or at least partially arrest the symptoms of the disease or condition. Amounts effective for this use will depend on the severity and course of the disease or condition, previous therapy, the patient's health status, weight, and response to the drugs, and the judgment of the treating physician.

In prophylactic applications, compositions containing the compounds described herein are administered to a patient susceptible to or otherwise at risk of a particular disease, disorder or condition. Such an amount is defined to be a "prophylactically effective amount or dose." In this use, the precise amounts also depend on the patient's state of health, weight, and the like. When used in a patient, effective amounts for this use will depend on the severity and course of the disease, disorder or condition, previous therapy, the patient's health status and response to the drugs, and the judgment of the treating physician.

### Treatments

In some aspects, the present disclosure provides a method of treating or preventing a disease in a subject in need thereof comprising administering a *N*-glycosylation pathway inhibitor to the subject in need thereof. In some embodiments, the disease is caused by a respiratory virus. In some embodiments, the method comprises administering a therapeutically effective amount of the *N*-glycosylation pathway inhibitor.

In some aspects, the present disclosure provides a method of inhibiting replication of a respiratory virus in a subject in need thereof, comprising administering a STT3A/STT3B inhibitor to the subject in need thereof. In some embodiments, the method comprises administering a therapeutically effective amount of the *N-*glycosylation pathway inhibitor.

In some aspects, the present disclosure provides a method of inhibiting replication of a respiratory virus in a subject in need thereof, the method comprising administering to the subject an *N-*glycosylation pathway inhibitor. In some embodiments, the present disclosure provide a method of treating a respiratory virus infection in a subject in need thereof, the method comprising administering to the subject an *N*-glycosylation pathway inhibitor. In some embodiments, the present disclosure provide a method of preventing a respiratory virus infection in a subject in need thereof, the method comprising administering to the subject an *N-*glycosylation pathway inhibitor. In some embodiments, the method comprises administering a therapeutically effective amount of the *N*-glycosylation pathway inhibitor.

In some embodiments, the respiratory virus is selected from an influenza virus, a rhinovirus, a coronavirus, a metapneumovirus, an adenovirus, a syncytial virus, a bocavirus, and a parainfluenza virus. In some embodiments, the respiratory virus is selected from an influenza virus, a rhinovirus and a parainfluenza virus. In some embodiments, the respiratory virus is selected from the group consisting of an influenza virus, a rhinovirus, and a parainfluenza virus. In some embodiments, the respiratory virus is an influenza virus. In some embodiments, the respiratory virus is not a coronavirus. In some embodiments, the respiratory virus is not a SARS-CoV-2 virus. In some embodiments, the respiratory virus is a coronavirus. In some embodiments, the coronavirus is a SARS-CoV-2 variant selected from: alpha variant, beta variant, delta variant, and omicron variant.

In some embodiments, the subject is a mammal. In some embodiments, the subject is a human.

### Pharmaceutical Formulations

In some aspects, the present disclosure provides a pharmaceutical composition comprising a STT3A/STT3B inhibitor and at least one pharmaceutically acceptable excipient.

A STT3A/STT3B inhibitor may be formulated in any suitable pharmaceutical formulation. A pharmaceutical formulation of the present disclosure typically contains an active ingredient (e.g., a STT3A/STT3B inhibitor), and one or more pharmaceutically acceptable excipients or carriers, including but not limited to: inert solid diluents and fillers, diluents, sterile aqueous solution and various organic solvents, permeation enhancers, antioxidents, solubilizers, and adjuvants. Preparations for such pharmaceutical composition are well-known in the art. See, e.g., Anderson, Philip O.; Knoben, James E.; Troutman, William G, eds., Handbook of Clinical Drug Data, Tenth Edition, McGraw-Hill, 2002; Pratt and Taylor, eds., Principles of Drug Action, Third Edition, Churchill Livingston, New York, 1990; Katzung, ed., Basic and Clinical Pharmacology, Ninth Edition, McGraw Hill, 2003; Goodman and Gilman, eds., The Pharmacological Basis of Therapeutics, Tenth Edition, McGraw Hill, 2001; Remingtons Pharmaceutical Sciences, 20th Ed., Lippincott Williams & Wilkins., 2000; Martindale, The Extra Pharmacopoeia, Thirty-Second Edition (The Pharmaceutical Press, London, 1999).

### EXAMPLES

### CRISPR Screen Study Identifying Antiviral Targets

Gene-knockout studies were conducted in cellular infection models of Influenza A (IAV) strain PR8, Human rhinovirus (HRV) strain 16, Parainfluenza virus 3 (PIV-3) strain JS, and human coronavirus 229E (CoV-229E). These studies, discussed in detail below, identified STT3A and STT3B are critical genes for viral replication. These experiments, the results of which are summarized in Table 1 below, demonstrate that inhibition of the OST complex is an effective treatment modality for inhibiting replication of respiratory viruses and treating and/or preventing viral infection, including a respiratory virus infection.

Genome-wide and subsequent subpool (smaller set of genes than full genome) pooled CRISPR knockout screens were performed in cellular infection models of Influenza A (IAV) strain PR8, Human rhinovirus (HRV) strain 16, Parainfluenza virus 3 (PIV-3) strain JS, and human coronavirus 229E (CoV-229E). The screening phenotypes were based on either virus-induced cell death or detection of a viral protein (such as IAV nucleoprotein) or reporter gene (such as a virus genome-encoded fluorescent GFP). Lentivirus-encoded CRISPR libraries were introduced into Cas9-expressing cells at low multiplicity of transduction such that each transduced cell was likely to receive one guide RNA targeting one gene for depletion. The pooled library of cells was infected with virus, and the population of cells was enriched for the screening phenotype (e.g., surviving cells, or FACS sorting of low and high levels of reporter gene). Upon sequencing of the abundance of guide RNAs in the enriched cell populations, MAGeCK analysis was performed to determine the statistical significance of gene enrichment. A gene was considered a screen hit if guide RNAs for the gene were associated with reduced infection with -log(p-value) > 3. Such a gene was thus identified as a "pro-viral" gene that promotes viral infection.

Additionally, select genes were targeted individually for depletion by introducing a single guide RNA, again followed by infection to assess the level of infection. Genes for which this targeted depletion resulted in statistically significant (p-value < 0.05) reduction of infection were also identified as pro-viral genes.

Table 1 summarizes of some of the results obtained from the CRISPR knockout screen. An "x" denotes that the experiment determined the knocked out gene is a "pro-viral" gene that promotes viral infection.

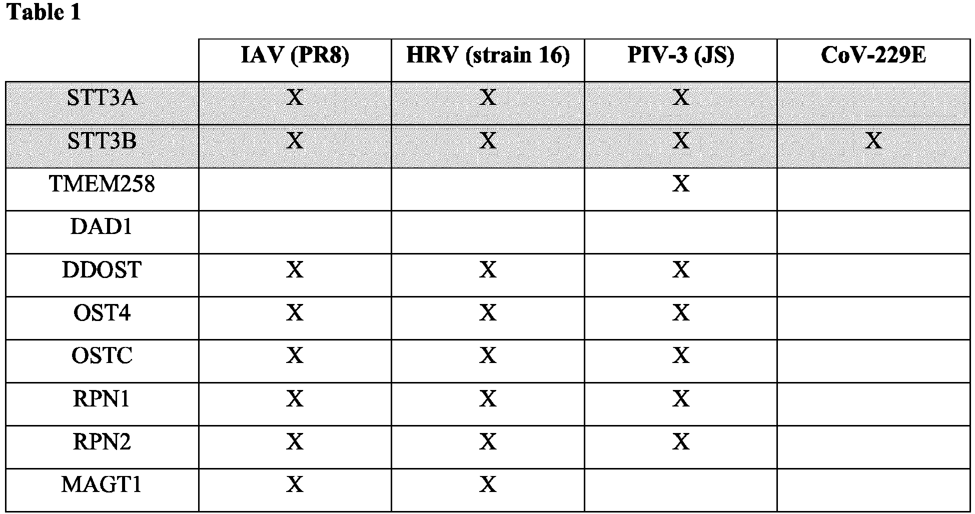

Table 1, above, indicates pro-viral genes identified as hits for each of the indicated viruses for genes in the oligosaccharyltransferase (OST) complex, which is an essential component of the N-glycosylation pathway. The OST complex shares most components but takes two forms, OST-A or OST-B, where STT3A is the catalytic subunit of OST-A, and STT3B is the catalytic subunit of OST-B. As the catalytic subunits of the OST complex, STT3A and STT3B (highlighted in grey in the table) represent potential targets for inhibition with the goal of reducing respiratory virus infection. The results indicate that inhibition of other components of the OST complex, or the OST complex as a whole, can reduce respiratory virus infection.

More broadly, the same analysis was applied to the major genes of the remainder of the N-glycosylation pathway. The results of this analysis is disclosed below in Table 2. Similar to the above, the indicated pro-viral hit genes represent potential targets for inhibition to reduce respiratory virus infection. The widespread nature of these hits also suggests that inhibition of the N-glycosylation pathway in general represents a strategy to reduce respiratory virus infection.

**Table 2**

| | **IAV (PR8)** | **HRV (strain 16)** | **PIV-3 (JS)** | **CoV-229E** |
|---|---|---|---|---|
| DHDDS | | | X | |
| NUS1 | | | X | |
| SRD5A3 | | | X | |
| DOLPP1 | X | X | X | |
| DOLK | | X | X | |
| DPAGT1 | | X | X | X |
| ALG13 | | | X | |
| ALG14 | | X | X | |
| MPI | X | X | X | |
| PMM2 | X | X | X | |
| GMPPA | | | | |
| GMPPB | X | X | X | |
| ALG1 | X | X | X | X |
| ALG2 | | X | X | |
| ALG11 | | | X | |
| RFT1 | | X | X | X |
| MPDU1 | X | X | X | |
| DPM1 | X | X | X | |
| DPM2 | X | X | X | |
| DPM3 | X | X | X | |
| ALG3 | X | X | X | |
| ALG9 | X | | X | |
| ALG12 | X | | X | |
| ALG5 | X | X | X | |
| ALG6 | X | X | X | |
| ALG8 | X | X | X | |
| ALG10 | | X | X | |
| MOGS | | X | X | |
| CALR | X | X | X | |
| CANX | | | | |
| PDIA3 | X | | | |
| GANAB | X | X | X | |
| MAN1B1 | | | X | |
| MAN1A1 | | | | |
| MAN1A2 | X | X | X | |
| MAN1C1 | | | | |
| MGAT1 | X | X | X | |
| MAN2A1 | | | | |
| MAN2A2 | | | | |
| MGAT2 | X | X | X | |
| FUT8 | | | | |
| B4GALT1 | | | | |
| ST6GAL1 | X | | | |
| ST6GAL2 | | | | |
| SLC35A3 | | | | |
| SLC39A8 | | | | |
| SLC35A2 | X | X | X | |
| SLC35C1 | | | | |
| SLC35A1 | X | X | X | |

In view of the gene knock-out studies disclosed above, it has been discovered that agents that modulate and/or inhibit the N-glycosylation pathway represents an effective strategy to treat respiratory virus infection and/or inhibit replication of respiratory viruses. Further, the gene knock-out studies disclosed above show that modulation and/or inhibition of the OST complex, STT3A and/or STT3B specifically is an effective strategy to treat respiratory virus infection and/or inhibit replication of respiratory viruses. In light of these results, it has been disclosed that STT3A/STT3B inhibitors are a promising treatment modality for treating and/or preventing respiratory virus infection.

The following numbered paragraphs set out preferred features and preferred combinations of features of the present invention.
1. A method of inhibiting replication of a respiratory virus in a subject in need thereof, the method comprising administering to the subject an *N-*glycosylation pathway inhibitor.
2. A method of treating or preventing a respiratory virus infection in a subject in need thereof, the method comprising administering to the subject an *N-*glycosylation pathway inhibitor.
3. The method of paragraphs 1 or 2, wherein the *N*-glycosylation pathway inhibitor is an OST complex inhibitor.
4. The method of paragraph 3, wherein the OST complex inhibitor is a small molecule OST complex inhibitor.
5. The method of paragraphs 1 or 2, wherein the *N*-glycosylation pathway inhibitor is selected from tunicamycin and NGI-1.
6. The method of paragraph 5, wherein the *N*-glycosylation pathway inhibitor is tunicamycin.
7. The method of paragraph 5, wherein the *N*-glycosylation pathway inhibitor is NGI-1.
8. The method of paragraph 3, wherein the OST complex inhibitor is STT3A/STT3B inhibitor.
9. The method of paragraph 8, wherein the STT3A/STT3B inhibitor is a small molecule STT3A/STT3B inhibitor.
10. The method of paragraph 8, wherein the STT3A/STT3B inhibitor comprises a protein.
11. The method of paragraph 10, wherein the STT3A/STT3B inhibitor comprises an antibody or antigen binding fragment thereof that selectively binds STT3A, STT3B, or both.
12. The method of any one of paragraphs 1 to 11, wherein the subject is a human.
13. The method of any one of paragraphs 1 to 12, wherein the respiratory virus is selected from the group consisting of: influenza virus, a rhinovirus, a coronavirus, a metapneumovirus, an adenovirus, a respiratory syncytial virus, a bocavirus, and a parainfluenza virus.
14. The method of any one of paragraphs 1 to 12, wherein the respiratory virus is selected from the group consisting of influenza virus, a rhinovirus, a metapneumovirus, an adenovirus, a respiratory syncytial virus, a bocavirus, and a parainfluenza virus.
15. The method of any one of paragraphs 1 to 12, wherein the respiratory virus is selected from the group consisting of an influenza virus, a rhinovirus, and a parainfluenza virus.
16. The method of any one of paragraphs 1 to 12, wherein the respiratory virus is not a coronavirus.
17. The method of any one of paragraphs 1 to 13, wherein the respiratory virus is not a SARS-CoV-2 virus.
18. The method of paragraph 13, wherein the coronavirus is a SARS-CoV-2 variant selected from: alpha variant, beta variant, delta variant, and omicron variant.
19. The method of any one of paragraphs 1 and 3 to 18, wherein the method comprises treating a respiratory virus infection in a subject in need thereof.
20. The method of any one of paragraphs 1 and 3 to 18, wherein the method comprises preventing a respiratory virus infection in a subject in need thereof.
21. An *N*-glycosylation pathway inhibitor for use in inhibiting replication of a respiratory virus in a subject in need thereof.
22. An *N-*glycosylation pathway inhibitor for use in treating or preventing a respiratory virus infection in a subject in need thereof.
23. The *N-*glycosylation pathway inhibitor of paragraphs 21 or 22, wherein the *N-*glycosylation pathway inhibitor is an OST complex inhibitor.
24. The *N-*glycosylation pathway inhibitor of paragraph 23, wherein the OST complex inhibitor is a small molecule OST complex inhibitor.
25. The *N-*glycosylation pathway inhibitor of paragraphs 21 or 22, wherein the *N-*glycosylation pathway inhibitor is selected from tunicamycin and NGI-1.
26. The *N-*glycosylation pathway inhibitor of paragraph 25, wherein the *N-*glycosylation pathway inhibitor is tunicamycin.
27. The *N-*glycosylation pathway inhibitor of paragraph 25, wherein the *N-*glycosylation pathway inhibitor is NGI-1.
28. The *N-*glycosylation pathway inhibitor of paragraph 23, wherein the OST complex inhibitor is STT3A/STT3B inhibitor.
29. The *N-*glycosylation pathway inhibitor of paragraph 28, wherein the STT3A/STT3B inhibitor is a small molecule STT3A/STT3B inhibitor.
30. The *N-*glycosylation pathway inhibitor of paragraph 28, wherein the STT3A/STT3B inhibitor comprises a protein.
31. The *N-*glycosylation pathway inhibitor of paragraph 30, wherein the STT3A/STT3B inhibitor comprises an antibody or antigen binding fragment thereof that selectively binds STT3A, STT3B, or both.
32. The *N*-glycosylation pathway inhibitor of any one of paragraphs 21 to 31, wherein the subject is a human.
33. The *N-*glycosylation pathway inhibitor of any one of paragraphs 21 to 32, wherein the respiratory virus is selected from the group consisting of influenza virus, a rhinovirus, a coronavirus, a metapneumovirus, an adenovirus, a respiratory syncytial virus, a bocavirus, and a parainfluenza virus.
34. The *N-*glycosylation pathway inhibitor of any one of paragraphs 21 to 32, wherein the respiratory virus is selected from the group consisting of influenza virus, a rhinovirus, a metapneumovirus, an adenovirus, a respiratory syncytial virus, a bocavirus, and a parainfluenza virus.
35. The *N-*glycosylation pathway inhibitor of any one of paragraphs 21 to 32, wherein the respiratory virus is selected from the group consisting of: an influenza virus, a rhinovirus, and a parainfluenza virus.
36. The *N-*glycosylation pathway inhibitor of any one of paragraphs 21 to 32, wherein the respiratory virus is not a coronavirus.
37. The *N*-glycosylation pathway inhibitor of any one of paragraphs 21 to 33, wherein the respiratory virus is not a SARS-CoV-2.
38. The *N*-glycosylation pathway inhibitor of paragraph 33, wherein the coronavirus is a SARS-CoV-2 variant selected from: alpha variant, beta variant, delta variant, and omicron variant.

## Claims

1. An *N*-glycosylation pathway inhibitor for use in a method of treating or preventing a respiratory virus infection in a subject in need thereof.

2. An *N*-glycosylation pathway inhibitor for use according to claim 1, which is for use by inhibiting replication of a respiratory virus in a subject in need thereof.

3. The *N*-glycosylation pathway inhibitor for use according to claim 1 or claim 2, wherein the *N*-glycosylation pathway inhibitor is an OST complex inhibitor.

4. The *N*-glycosylation pathway inhibitor for use according to claim 3, wherein the OST complex inhibitor is a small molecule OST complex inhibitor.

5. The *N*-glycosylation pathway inhibitor for use according to claim 1 or claim 2, wherein the *N*-glycosylation pathway inhibitor is selected from tunicamycin and NGI-1.

6. The *N*-glycosylation pathway inhibitor for use according to claim 3, wherein the OST complex inhibitor is STT3A/STT3B inhibitor, and wherein the STT3A/STT3B inhibitor is optionally a small molecule STT3A/STT3B inhibitor.

7. The *N*-glycosylation pathway inhibitor for use according to claim 6, wherein the STT3A/STT3B inhibitor comprises a protein.

8. The *N*-glycosylation pathway inhibitor for use according to claim 7, wherein the STT3A/STT3B inhibitor comprises an antibody or antigen binding fragment thereof that selectively binds STT3A, STT3B, or both.

9. The *N*-glycosylation pathway inhibitor for use according to any one of claims 1-8, wherein the subject is a human.

10. The *N*-glycosylation pathway inhibitor for use according to any one of claims 1-9, wherein the respiratory virus is selected from the group consisting of: influenza virus, a rhinovirus, a coronavirus, a metapneumovirus, an adenovirus, a respiratory syncytial virus, a bocavirus, and a parainfluenza virus.

11. The *N-*glycosylation pathway inhibitor for use according to any one of claims 1-9, wherein the respiratory virus is selected from the group consisting of: influenza virus, a rhinovirus, a metapneumovirus, an adenovirus, a respiratory syncytial virus, a bocavirus, and a parainfluenza virus.

12. The *N-*glycosylation pathway inhibitor for use according to any one of claims 1-9, wherein the respiratory virus is selected from the group consisting of: an influenza virus, a rhinovirus, and a parainfluenza virus.

13. The *N-*glycosylation pathway inhibitor for use according to any one of claims 1-9, wherein the respiratory virus is not a coronavirus.

14. The *N*-glycosylation pathway inhibitor for use according to any one of claims 1-10, wherein the respiratory virus is not a SARS-CoV-2.

15. The *N*-glycosylation pathway inhibitor for use according to claim 10, wherein the coronavirus is a SARS-CoV-2 variant selected from: alpha variant, beta variant, delta variant, and omicron variant.
